# EUROPÄISCHE PATENTSCHRIFT

(11) **EP 0 863 724 B1**
(45) Veröffentlichungstag und Bekanntmachung des Hinweises auf die Patenterteilung: **02.06.1999**
(21) Anmeldenummer: 97903238.0
(22) Anmeldetag: 07.02.1997
(51) Int. Cl.: A61B 17/04, A61B 17/86

(54) **KNOCHENSCHRAUBE**
BONE SCREW
VIS A OS

(30) Priorität: 28.02.1996 DE 19607517
(43) Veröffentlichungstag der Anmeldung: 16.09.1998
(73) Patentinhaber: Biedermann, Lutz, 78048 Villingen-Schwenningen (DE); Harms, Jürgen, 76337 Waldbronn (DE)
(72) Erfinder: Biedermann, Lutz, 78048 Villingen-Schwenningen (DE); Harms, Jürgen, 76337 Waldbronn (DE)
(74) Vertreter: Prüfer, Lutz H., Dipl.-Phys.
(86) Internationale Anmeldenummer: EP9700567
(87) Internationale Veröffentlichungsnummer: WO9731574

(56) Entgegenhaltungen:
- EP-A- 0 504 915
- DE-A- 3 936 703

## Beschreibung

Die Erfindung betrifft eine Knochenschraube nach dem Oberbegriff des Patentanspruches 1.

Eine derartige Knochenschraube ist aus der DE 39 36 703 C2 bekannt. Bei dieser Knochenschraube wird das vordere freie Ende des Gewindeschaftes der Schraube mittels eines in eine Längsbohrung der Schraube eingeführten Spreizteiles aufgespreizt. Diese Aufspreizung dient einmal als Rückdrehsicherung und zum anderen zur Erhöhung der Verankerungskräfte zwischen dem Knochenmaterial und dem Schraubengewinde.

Der dafür erforderliche Verschraubmechanismus macht eine Mindestabmessung erforderlich.

Es ist Aufgabe der Erfindung, eine Knochenschraube mit besonders kleinem Durchmesser mit Rückdrehsicherung und Verankerungsmöglichkeit im Knochen zu schaffen, welche nach ihrem Einschrauben in den Knochen in einem zweiten Arbeitsgang fein dosiert an der Spitze aufgespreizt werden kann.

Die Aufgabe wird durch eine Knochenschraube nach Patentanspruch 1 gelöst.

Weiterbildungen der Erfindung sind in den Unteransprüchen angegeben.

Ausführungsbeispiele der Erfindung werden im folgenden anhand der Figuren näher beschrieben.

Von den Figuren zeigen:
- Fig. 1: den Gewindeschaftteil mit Kopf der Knochenschraube;
- Fig. 2: das Spreizteil der Knochenschraube;
- Fig. 3: die Knochenschraube in zusammengesetztem Zustand vor dem Einschrauben in teilweise geschnittener Darstellung;
- Fig. 4: eine vergrößerte Darstellung der Spitze der Knochenschraube von Fig. 3 in teilweise geschnittener Darstellung;
- Fig. 5: die Knochenschraube in eingeschraubtem Zustand mit aufgespreiztem vorderen Ende in teilweise geschnittener Darstellung und
- Fig. 6: eine vergrößerte Darstellung des aufgespreizten vorderen Endes der Knochenschraube von Fig. 5.

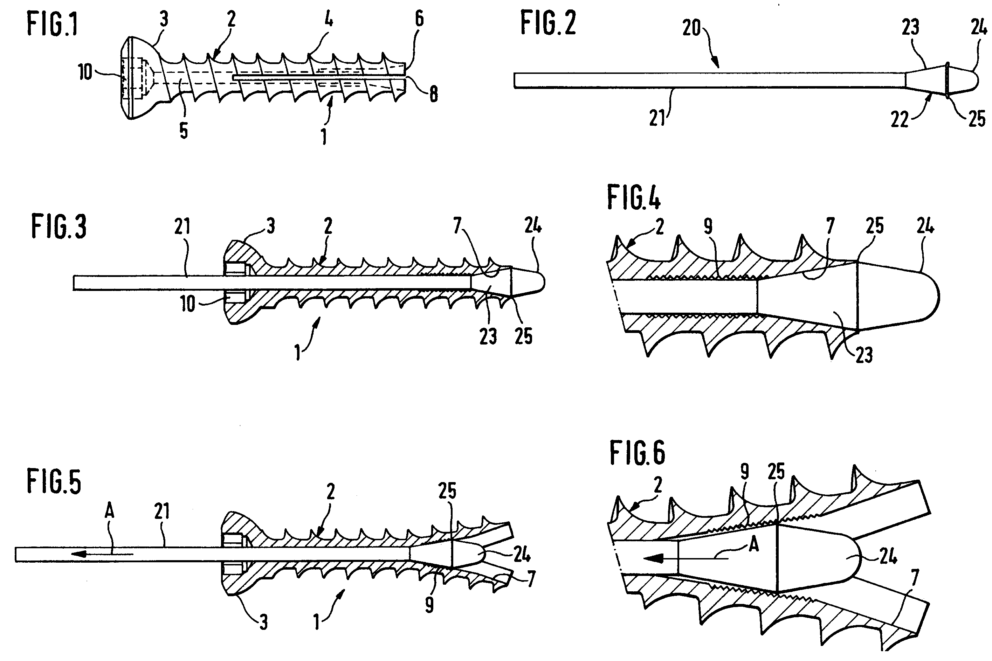

Wie aus den Figuren 1 bis 4 ersichtlich ist, weist die Knochenschraube 1 einen Gewindeschaftteil 2 und einen damit einstückig ausgebildeten Kopf 3 auf. Der Gewindeschaftteil 2 weist ein üblicherweise für Knochenschrauben verwendetes Außengewinde 4 auf. Wie insbesondere aus Fig. 1 ersichtlich ist, erstreckt sich durch das Innere der Knochenschraube 1 eine koaxial ausgerichtete Längsbohrung 5.

Diese weist an ihrem dem Kopf 3 abgewandten freien Ende 6 eine sich zu dem freien Ende 6 hin erweiternde koaxial angeordnete konische Ausnehmung 7 auf. In der Wandung des Gewindeschaftteiles 2 sind von dem dem Kopf 3 abgewandten freien Ende 6 ausgehend sich parallel zur Längsachse des Gewindeschaftteiles 2 erstreckende Schlitze 8 vorgesehen. Diese Schlitze 8 erstrecken sich über eine Länge, welche etwas mehr als die Hälfte der Länge des Gewindeschaftes 2 ist. Mindestens ist die Länge der Schlitze jedoch gleich der zweifachen Länge in axialer Richtung der konischen Ausnehmung 7. Vorzugsweise ist ein Paar solcher Schlitze vorgesehen, die um 180° gegeneinander versetzt angeordnet sind.
In einem an die konische Ausnehmung 7 angrenzenden Abschnitt der Längsbohrung 5 weist die Oberfläche einen im wesentlichen sägezahnförmigen Abschnitt 9 auf. Der sägezahnförmige Abschnitt 9 wird durch in Umfangsrichtung vorgesehene Einkerbungen in die Innenwand des Gewindeschaftteiles gebildet, wobei die durch die Einkerbung gebildeten Zähne jeweils eine dem Kopf 3 zugewandte und eine dem freien Ende 6 zugewandte Flanke aufweisen. Die dem Kopf 3 zugewandte Flanke ist dabei in nichtaufgespreiztem Zustand des Gewindeschaftteiles 2 annähernd senkrecht zur Längsachse des Gewindeschaftteiles ausgerichtet, während die dem freien Ende 6 zugewandte Flanke der Zähne in einem spitzen Winkel zur Längsachse ausgerichtet ist. Der sägezahnförmige Abschnitt 9 erstreckt sich über eine Länge in axialer Richtung der Schraube, die wenigstens gleich der sich in axialer Richtung ergebenden Länge der konischen Ausnehmung 7 ist.

Der Kopf 3 weist auf seiner dem freien Ende 6 gegenüberliegenden Seite in axialer Richtung gesehen eine Ausnehmung 10 auf, die so ausgebildet ist, daß die Knochenschraube 1 mittels dieser Öffnung und eines damit zusammenwirkenden Schraubendrehers in einen Knochen eindrehbar ist. Die Ausnehmung 10 weist beispielsweise eine Sechskantform auf zum Ineingriffbringen mit einem Sechskant-Schraubendreher.

Es ist ein als Zugstange dienendes Spreizteil 20 vorgesehen, welches aus einem stabförmigen Schaft 21 mit einem an einem Ende desselben gebildeten Spitzenteil 22 besteht. Der Durchmesser des Schaftes 21 ist so bemessen, daß der Schaft 21 in der Längsbohrung 5 der Knochenschraube zwar geführt, aber doch in axialer Richtung verschiebbar ist. Die Länge des Schaftes 21 ist umsoviel größer als die Länge der Knochenschraube 1 in axialer Richtung, daß der Schaft 21 in in die Schraube eingesetztem Zustand aus dem Kopf 3 heraussteht und eine genügende Angriffsfläche für ein damit zusammenwirkendes Werkzeug bietet. Das Spitzenteil 22 weist auf seiner an dem Schaft 21 angrenzenden Seite eine Außenwandung mit einem konischen Abschnitt 23 auf, der zu dem Schaft 21 hin verjüngt verläuft und in seinen Abmessungen so ausgebildet ist, daß er in die konische Ausnehmung 7 formschlüssig hineinpaßt. An seiner dem Schaft 21 und damit auch dem Kopf 3 der Knochenschraube 1 abgewandten Seite weist das Spitzenteil 22 eine die Spitze der Knochenschraube bildende Spitze 24 auf. Die Spitze 24 ist ebenfalls konisch ausgebildet, wobei sie sich zu der dem Kopf 3 abgewandten Seite hin verjüngt. Das freie Ende der Spitze 24 ist abgerundet. In axialer Richtung gesehen etwa mittig weist das Spitzenteil 22 eine in Umfangsrichtung vorgesehene Kante 25 auf, deren Durchmesser etwas größer ist als der Durchmesser der konischen Ausnehmung 7 am freien Ende 6. Die Kante 25 wird dadurch gebildet, daß die Spitze 24 unmittelbar angrenzend an den konischen Abschnitt 23 des Spitzenteils 22 einen geringeren Durchmesser hat, als der konische Abschnitt 23, so daß dieser einen Vorsprung bildet. Die Abmessung der Kante 25 ist so, daß die Kante 25 in den Zähnen des sägezahnförmigen Abschnittes 9 einrasten kann, wenn das Spreizteil in den Gewindeschaftteil eingesetzt ist und in Richtung des Kopfes gezogen wird.

Ein geeigneter Schraubendreher zum Eindrehen der Schraube weist eine zentrale Längsbohrung zum Hindurchführen des Schaftes 21 des Spreizteiles 20 auf.

In Betrieb wird zunächst der Schaft 21 vom freien Ende 6 des Gewindeschaftteiles her in die Längsbohrung 5 hineingeschoben, bis der konische Abschnitt 23 des Spitzenteils 22 in der konischen Ausnehmung 7 ruht und die Knochenschraube die übliche Spitze 24 in der aus den Figuren 3 und 4 ersichtlichen Weise aufweist. Das der Spitze 24 abgewandte Ende des Schaftes 21 steht dabei aus dem Kopf 3 der Schraube heraus. Das Eindrehen der Schraube erfolgt nun in der bekannten Art mittels des Schraubeneindrehers.

Wie insbesondere aus den Figuren 5 und 6 ersichtlich ist, erfolgt, nachdem die Knochenschraube 1 exakt positioniert ist, ein Aufspreizen des freien Endes des Gewindeschaftteiles 2 durch Ausübung von Zugkräften auf den Schaft 21 des Spreizteiles 20 in Richtung des Pfeiles A von Fig. 5. Die Zugkräfte auf den Schaft 21 werden durch Eingreifen des freien Abschnittes mit einer Art Nietenzieher ausgeübt, der am Schraubenkopf 3 abgestützt wird. Dabei wird das Spitzenteil 22 so in die Längsbohrung 5 des Gewindeschaftteiles 2 gezogen, daß sich die Kante 25 in den Zähnen des Abschnittes 9 verkrallt. Dabei erfolgt eine wohldosierte Aufspreizung des geschlitzten Abschnittes. Nachdem der Aufspreizvorgang beendet ist, wird der überstehende Teil des Schaftes 21 abgeschnitten.

Der Abschnitt 9 muß nicht notwendigerweise sägezahnförmig ausgebildet sein. Die Einkerbung kann auch so ausgebildet sein, daß die Oberfläche der Zähne gekrümmt ist. Entscheidend ist, daß die Steigung der Flanken so bemessen ist, daß in aufgespreiztem Zustand ein selbständiges Lösen des Spitzenteiles 22 aus der Einkerbung nicht möglich ist.

## Patentansprüche

1. Knochenschraube mit
einem Gewindeschaftteil (2) und einem Kopf (3),
einer sich entlang der Längsachse des Gewindeschaftteiles (2) erstreckenden Längsbohrung (5),
einem sich zu dem dem Kopf (3) abgewandten freien Ende (6) des Gewindeschaftteiles (2) erweiternden Abschnitt (7) der Längsbohrung (5),
wenigstens zwei sich in diesem Abschnitt (7) befindlichen und parallel zur Längsachse erstreckenden Schlitzen (8) in dem Gewindeschaftteil (2), und
einem Spreizteil (20), welches aus einem die Spitze der Knochenschraube bildenden Spitzenteil (22) und einem sich in die Längsbohrung (5) erstreckenden Schaft (21) gebildet ist,
gekennzeichnet durch
eine an dem Spitzenteil (22) und dem Gewindeschaftteil (2) vorgesehene Arretierung (9, 25) zum Arretieren des Spitzenteiles (22) in dem Gewindeschaftteil (2).

2. Knochenschraube nach Anspruch 1, dadurch gekennzeichnet, daß die Arretierung aus einer an der Innenwand des Gewindeschaftteiles (2) vorgesehenen Einkerbung (9) und einem an dem Spitzenteil (22) vorgesehenen und in die Einkerbung einrastenden Vorsprung (25) gebildet ist.

3. Knochenschraube nach Anspruch 1 oder 2, dadurch gekennzeichnet, daß das Spreizteil (20) einstückig ausgebildet ist, wobei das Spitzenteil (22) an einem Ende des Schaftes (21) vorgesehen ist.

4. Knochenschraube nach einem der Ansprüche 1 bis 3, dadurch gekennzeichnet, daß der sich zum freien Ende (6) hin erweiternde Abschnitt (7) der Längsbohrung (5) im wesentlichen konisch ausgebildet ist.

5. Knochenschraube nach einem der Ansprüche 1 bis 4, dadurch gekennzeichnet, daß das Spitzenteil (22) einen ersten sich zum Schaft (21) hin verjüngenden im wesentlichen konischen Abschnitt (23) und angrenzend daran einen zweiten, sich in Richtung der Spitze der Schraube hin verjüngenden Abschnitt (24) aufweist, wobei der konische Abschnitt (23) in den erweiterten Abschnitt (7) der Längsbohrung (5) formschlüssig hineinpaßt.

6. Knochenschraube nach Anspruch 4 oder 5, dadurch gekennzeichnet, daß der Gewindeschaftteil (2) angrenzend an den sich erweiternden Abschnitt (7) an seiner Innenwand die Einkerbung (9) aufweist, und das Spitzenteil (22) an seiner den größten Durchmesser senkrecht zur Längsachse der Schraube aufweisenden Stelle den Vorsprung (25) in radialer Richtung aufweist, der mit der Einkerbung (9) in Eingriff gelangt.

7. Knochenschraube nach einem der Ansprüche 2 bis 6, dadurch gekennzeichnet, daß der Vorsprung (25) durch eine in Umfangsrichtung an dem Spitzenteil (22) vorgesehene und in radialer Richtung hervorstehende Kante (25) gebildet ist.

8. Knochenschraube nach einem der Ansprüche 2 bis 7, dadurch gekennzeichnet, daß die Einkerbung (9) als sägezahnförmiger Abschnitt ausgebildet ist.

## Claims

1. A bone screw with
a threaded shaft component (2) and a head (3),
a longitudinal bore (5) extending along the longitudinal axis of the threaded shaft component (2),
a section (7) of the longitudinal bore (5) which widens towards the free end (6) of the threaded shaft component (2) facing away from the head (3),
at least two slits (8) in the threaded shall component (2) which are located in this section (7) and extend parallel to the longitudinal axis, and
a spreading component (20) which is formed from a tip component (22) forming the tip of the bone screw and a shaft (21) extending into the longitudinal bore (5),
characterized by
a stop device (9, 25) provided on the tip component (22) and the threaded shaft component (2) for stopping the tip component (22) in the threaded shaft component (2).

2. A bone screw according to claim 1, characterized in that the stop device is formed from a notching (9) provided on the internal wall of the threaded shaft component (2) and a projection (25) which is provided on the tip component (22) and latches into the notching.

3. A bone screw according to claim 1 or 2, characterized in that the spreading component (20) is constructed in one piece, the tip component (22) being provided on one end of the shaft (21).

4. A bone screw according to one of claims 1 to 3 characterized in that the section (7) of the longitudinal bore (5) which widens towards the free end (6) is substantially conical in construction.

5. A bone screw according to one of claims 1 to 4, characterized in that the tip component (22) has a first section (23) which narrows towards the shall (21) and is substantially conical and a second section (24) adjacent thereto which narrows in the direction of the tip of the screw, the conical section (23) fitting into the widened section (7) of the longitudinal bore (5) in form-fitting manner.

6. A bone screw according to claim 4 or 5, characterized in that the threaded shaft component (2) has the notching (9) on its internal wall adjacent to the widening section (7), and the tip component (22) has, in the radial direction, at its position having the largest diameter perpendicular to the longitudinal axis of the screw, the projection (25) which engages with the notching (9).

7. A bone screw according to one of claims 2 to 6, characterized in that the projection (25) is formed by an edge (25) which is provided on the tip component (22) in the circumferential direction and projects in the radial direction.

8. A bone screw according to one of claims 2 to 7, characterized in that the notching (9) is constructed as a sawtooth-like section.

## Revendications

1. Vis à os comportant une partie (2) formant tige filetée et une tête (3) ; un trou longitudinal (5) s'étendant le long de l'axe longitudinal de la partie (2) formant tige filetée ; un segment (7) du trou longitudinal (5) s'étendant vers l'extrémité libre (6), opposée à la tête (3), de la partie (2) formant tige filetée ; au moins deux fentes (8) dans la partie (2) formant tige filetée, se trouvant dans ce segment (7) et s'étendant parallèlement à l'axe longitudinal ; et une pièce formant écarteur (20), qui est constituée d'une partie (22) formant pointe, qui forme la pointe de la vis à os, et d'une tige (21), s'étendant dans le trou longitudinal (5) ; caractérisée par un blocage (9, 25), prévu au niveau de la partie (22) formant pointe et de la partie (2) formant tige filetée, pour bloquer la partie (22) formant pointe dans la partie (2) formant tige filetée.

2. Vis à os selon la revendication 1, caractérisée en ce que le blocage est constitué d'une entaille (9) prévue sur la paroi intérieure de la partie (2) formant tige filetée et d'une saillie (25) prévue sur la partie (22) formant pointe et entrant en prise avec l'entaille.

3. Vis à os selon la revendication 1 ou 2, caractérisée en ce que la pièce (20) formant écarteur a une structure monobloc, la partie (22) formant pointe étant prévue à une extrémité de la tige (21).

4. Vis à os selon l'une des revendications 1 à 3, caractérisée en ce que le segment (7) du trou longitudinal (5), qui s'élargit vers l'extrémité libre (6) a une forme sensiblement conique.

5. Vis à os selon l'une des revendications 1 à 4, caractérisée en ce que la partie (22) formant pointe comporte un premier segment (23), sensiblement conique, se rétrécissant vers la tige (21), et, contigu à ce dernier, un deuxième segment (24) qui se rétrécit dans la direction de la pointe de la vis, le segment conique (23) étant ajusté par une liaison avec correspondance de forme dans le segment élargi (7) du trou longitudinal (5).

6. Vis à os selon la revendication 4 ou 5, caractérisée en ce que la partie (2) formant tige filetée présente l'entaille (9) sur sa paroi intérieure, en un point contigu au segment (7) qui va s'élargissant, et la partie (22) formant pointe présente la saillie (25) dans la direction radiale, en son point présentant le plus grand diamètre perpendiculairement à l'axe longitudinal de la vis, ladite saillie entrant en prise avec l'entaille (9).

7. Vis à os selon l'une des revendications 2 à 6, caractérisée en ce que la saillie (25) est formée par une arête (25) prévue sur la circonférence de la partie (22) formant pointe et faisant saillie dans la direction radiale.

8. Vis à os selon l'une des revendications 2 à 7, caractérisée en ce que l'entaille est constituée d'une partie en dents de scie.
